# EUROPEAN PATENT APPLICATION

(11) **EP 3 640 646 A1**
(43) Date of publication of application: **22.04.2020**
(21) Application number: 18200635.3
(22) Date of filing: 16.10.2018
(51) Int. Cl.: G01N 35/00

(54) **METHOD OF OPERATING AN ANALYTICAL LABORATORY**

(71) Applicant: F. Hoffmann-La Roche AG, 4070 Basel (CH); Roche Diagnostics GmbH, 68305 Mannheim (DE)
(72) Inventor: FRENZEL, Matthias, 68305 Mannheim (DE); MAETZLER, Marco, 6343 Rotkreuz (CH); McCAUGHEY, Andrew, 6343 Rotkreuz (CH)
(74) Representative: Gyenge, Zoltán

(57) **Abstract**

Disclosed is a method of operating an analytical laboratory, comprising: receiving and identifying a biological sample by a pre-analytical laboratory instrument; retrieving corresponding test order(s); determining a target laboratory instrument for each of the test order(s); determining whether the target laboratory instrument(s) is ready to carry out the test order; transmitting a readiness command to the target laboratory instrument(s) if the target laboratory instrument is not ready; executing instructions of the readiness command by the target laboratory instrument; instructing the target laboratory instrument(s) to process the biological sample according to the test order if the target laboratory instrument is ready to carry out the test order and the target laboratory instrument(s) processing the biological sample according to the test order as instructed.

## Description

### TECHNICAL FIELD

The present application relates to a computer implemented method of operating an analytical laboratory, in particular an in-vitro diagnostic laboratory. The application further relates to an I analytical laboratory configured to carry out the disclosed method. The application further relates to a computer program product comprising instructions which, when executed by a control unit of an analytical laboratory causes it to carry out the disclosed method.

### BACKGROUND

In vitro diagnostic testing has a major effect on clinical decisions, providing physicians with pivotal information. In analytical laboratories, in particular, in-vitro diagnostic laboratories, a multitude of analyses on biological samples are executed by laboratory instruments in order to determine physiological and biochemical states of patients, which can be indicative of a disease, nutrition habits, drug effectiveness, organ function and the like.

According to established laboratory procedures in complex analytical laboratories, a plurality of instruments process biological samples according to test orders, each test order defining one or more processing steps to be carried out on the biological sample. After the biological sample has been received and identified by a pre-analytical laboratory instrument, a control unit retrieves the corresponding test orders and determines which instruments (referred hereafter as target instrument(s)) are required to process the biological sample according to the test order(s). Having identified the target instrument(s), the control unit instructs the target laboratory instrument(s) to process the biological sample according to the test order(s).

It has been observed that significant delays occur between receipt respectively processing of the biological sample by the target laboratory instrument(s).

Such delays considerably affect the turn-around time of biological samples, that is the time between receipt of a biological sample and completion of the corresponding test order(s).

Hence there is a need for a method of operating an analytical laboratory, respectively an analytical laboratory system to provide reduced and/ or predicable Turn-Around-Times TAT for processing biological samples.

### SUMMARY

Applicant has observed that such delays are caused by the laboratory instruments not being ready to process the biological sample at the time the sample is loaded into them.

Embodiments herein disclosed address the above-identified need by a method of operating an analytical laboratory, comprising the steps of:
a) receiving and identifying a biological sample by a pre-analytical laboratory instrument of the analytical laboratory;
b) retrieving an order list from a database, the order list comprising one or more test order(s) defining at least one processing step to be carried out on the biological sample;
c) determining a target laboratory instrument of the analytical laboratory for each of the test order(s) which is configured to carry out the at least one processing step according to the test order;
d) determining whether the target laboratory instrument(s) is ready to carry out the test order;
e) transmitting a readiness command to the target laboratory instrument(s) if the target laboratory instrument(s) is not ready to carry out the test order, the readiness command comprising an instruction, which - when executed by the target laboratory instrument - causes the target laboratory instrument to become ready to carry out the test order;
f) executing instructions of the readiness command by the target laboratory instrument in order to cause the target laboratory instrument to become ready to carry out the test order;
g) instructing the target laboratory instrument(s) to process the biological sample according to the test order if the target laboratory instrument is ready to carry out the test order;
h) the target laboratory instrument(s) processing the biological sample according to the test order as instructed.

In order to save on running costs, in particular electricity, laboratory instruments or certain units thereof switch into low power modes, such as stand-by or hibernation modes or even completely shut down when not in use. However, waking up the laboratory instruments from such low power modes takes considerable amount of time. It has been observed that as a consequence, processing of a biological sample by a laboratory instrument(s) which is in a low power mode is often delayed as the biological sample is waiting idle during the readiness time of the laboratory instrument. Such occurrences lead to increased, respectively unpredictable Turn-Around-Times TAT.

In order to avoid biological samples having to sit idle while laboratory instrument(s) wake up from low power modes, according to embodiments disclosed herein, it is determined whether the instrument is powered on and not in a low-power mode. If the instrument is not powered on or in a low-power mode, a readiness command is sent to analytical instruments as soon as the biological sample has been identified and the corresponding test order(s) retrieved, the readiness command comprising instructions, which - when executed by the target laboratory instrument - causes the powering on respectively waking up of the target laboratory instrument from a low-power mode.

Besides instruments entering low power modes of operation, unavailability of all modules of the target instrument required to carry out the respective test order also leads to delays. There are multiple causes for modules of a target laboratory instrument not being operational, such as maintenance activates. To avoid delays caused by modules of the target laboratory instrument not being operational, according to embodiments disclosed herein, if one or more consumables required to carry out the respective test order are not available to the target laboratory instrument, a readiness command is sent to analytical instruments, the readiness command comprising instructions, which - when executed by the target laboratory instrument - causes all modules of the analytical instrument required to carry out the respective test order to be brought into operational state. According to embodiments disclosed herein, modules are made operational by interrupting or finalizing maintenance activities of the respective module. Alternatively, or additionally, according to further embodiments disclosed herein, modules of the target instrument are made operational by heating up of an incubation module respectively cooling down of a refrigeration module of the target instrument.

In a further scenario, unavailability of consumables required to carry out the respective test order leads to delays in processing of the biological sample. To avoid delays caused by consumables required to carry out the respective test order not being available, according to embodiments disclosed herein, it is determined whether all consumables required to carry out the respective test order are available. Corresponding, if one or more consumables required to carry out the respective test order are not available to the target laboratory instrument, a readiness command is sent to analytical instruments as soon as the biological sample has been identified and the corresponding test order(s) retrieved, the readiness command comprising instructions, which - when executed by the target laboratory instrument - causes all consumables required to carry out the respective test order to be made available to the target laboratory instrument. According to embodiments disclosed herein, consumables can be made available to the target laboratory instrument by loading - in particular automatic loading - the required consumable(s) from a storage location, such as a refrigerated storage unit. Alternatively, or additionally, according to further embodiments disclosed herein, consumable(s) are made available by carrying out consumable preparation steps, such as reconstitution of lyophilized reagents (freeze drying the liquid reagent which removes the liquid component and leaves a dry powder behind).

In an even further scenario, expired and or out of range quality control and/or calibration values lead to delays in processing of the biological sample, since such quality control and/or calibration processes take a certain amount of time and only thereafter can the biological sample be processed. To avoid delays caused by out of range quality control and/or calibration values, according to embodiments disclosed herein, if one or more quality control and/or calibration values are out of range or expired, a readiness command is sent to analytical instruments, the readiness command comprising instructions, which - when executed by the target laboratory instrument - causes carrying out of quality control and/or calibration steps to ensure all quality control and/or calibration values of the target laboratory instrument are up-to-date and valid.

According to further embodiments disclosed herein, the readiness command to the analytical instrument(s) is timed such that the analytical instrument(s) becomes ready/ ready just by the time the biological sample needs to be processed by the respective analytical instrument. This feature shall be referred to as just-in-time availability of the laboratory instruments.

According to even further embodiments disclosed herein, a readiness response from the target laboratory instruments is evaluated and an alternative analytical instrument is determined, if the readiness response is indicative that he target laboratory instrument is unable become ready up to process the biological sample according to the corresponding test order.

According to even further embodiments disclosed herein, the order of processing of a plurality of test orders corresponding to a biological sample is dynamically adjusted as a reaction to readiness responses from the analytical instruments to ensure fastest Turn-Around-Times TAT and/or most intensive use of low power modes of the instruments.

Embodiments disclosed herein are advantageous as they allow a significant reduction of Turn-Around-Times TAT of processing biological samples. The disclosed method/ system is proactive by sending a readiness command to instruments that will have to process the biological sample based on test order(s) as compared to known methods/ laboratory systems which are at best reactive by getting instruments ready only as a response to an incoming biological sample.

### BRIEF DESCRIPTION OF THE DRAWINGS

Further characteristics and advantages of the disclosed method / device /system will in the following be described in detail by means of the description and by making reference to the following drawings:
- Fig. 1: A flowchart illustrating a first embodiment of a herein disclosed method of operating an analytical laboratory;
- Fig. 2: A flowchart illustrating a further embodiment of a herein disclosed method of operating an analytical laboratory comprising a pre-analytical and an analytical laboratory instrument;
- Fig. 3: A flowchart illustrating a further embodiment of a herein disclosed method of operating an analytical laboratory further comprising a sample transportation system;
- Fig. 4: A flowchart illustrating a further embodiment of the method disclosed herein further comprising a step confirming execution of readiness command by the target instrument(s);
- Fig. 5A: First page of a flowchart illustrating further embodiments of a herein disclosed method of operating an analytical laboratory comprising a plurality of analytical laboratory instruments;
- Fig. 5B: Second page of a flowchart illustrating further embodiments of a herein disclosed method of operating an analytical laboratory comprising a plurality of analytical laboratory instruments;
- Fig. 5C: Second page of a flowchart illustrating further embodiments of a herein disclosed method wherein the sample workflow is adapted as a reaction to availability respectively readiness time of an analytical laboratory instrument;
- Fig. 5D: Second page of a flowchart illustrating further embodiments of a herein disclosed method wherein the readiness of a particular analytical laboratory instrument is delayed in view of processing time of the sample prior to processing by the particular analytical laboratory;
- Fig. 6: A flowchart illustrating a further embodiment of the method disclosed herein further comprising a shutdown of idle analytical instrument(s);
- Fig. 7: A highly schematic block diagram of an embodiment of the disclosed analytical laboratory;
- Fig. 8: A highly schematic block diagram of an embodiment of a pre-analytical laboratory instrument of the disclosed laboratory system;
- Fig. 9: A highly schematic block diagram of a further embodiment of a pre-analytical laboratory instrument of the disclosed laboratory system;
- Fig. 10: A highly schematic block diagram of an embodiment of an analytical laboratory instrument of the disclosed laboratory system;
- Fig. 11: A highly schematic block diagram of an embodiment of a post-analytical laboratory instrument of the disclosed laboratory system.

### DETAILED DESCRIPTION

Certain terms will be used in this patent application, the formulation of which should not be interpreted to be limited by the specific term chosen, but as to relate to the general concept behind the specific term.

The terms 'sample', patient sample' and 'biological sample' refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The term 'analyte' is a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Thus 'analyte' is a general term for substances for which information about presence and/or concentration is intended. Examples of analytes are e.g. glucose, coagulation parameters, endogenic proteins (e.g. proteins released from the heart muscle), metabolites, nucleic acids and so on.

The term 'analysis or 'analytical test' as used herein encompasses a laboratory procedure characterizing a parameter of a biological sample for qualitatively assessing or quantitatively measuring the presence or amount or the functional activity of an analyte.

The term 'reagent' as used herein refers to materials necessary for performing an analysis of analytes, including reagents for sample preparation, control reagents, reagents for reacting with the analyte to obtain a detectable signal, and/or reagents necessary for detecting the analyte. Such reagents may include reagents for isolating an analyte and/or reagents for processing a sample and/or reagents for reacting with an analyte to obtain a detectable signal and/or washing reagents and/or diluents.

The term 'reagent cassette' as used herein refers to any vessel/ container comprising a liquid or suspension of reagents. Alternatively, a reagent cassette is a holder for holding container(s) comprising a liquid or a suspension of reagents.

The term 'Quality control' or 'analytical quality control' refers to all those processes and procedures designed to ensure that the results of laboratory analysis (analytical tests) are consistent, comparable, accurate and within specified limits of precision.

The term 'quality control' QC materials as used herein refer to any composition with known concentration of an analyte, such as positive and negative controls, that serve the purpose of providing evidence that an analytical test is successfully performed and is giving the expected level of sensitivity and specificity as characterized during technical optimization and validation of the analytical test for diagnostic use. In other words, a 'quality control' is used in the present disclosure as referring to a physical sample used in one or several monitoring processes to monitor the performance of particular tests or assays of an analyzer. Positive controls primarily monitor calibration of the system and sensitivity. Negative controls are primarily used to evaluate the specificity of the analytical tests to identify false-positive results.

The terms 'sample container' and 'sample tube' refers to any individual container for storing, transporting, and/or processing a sample. In particular, said term without limitation refers to a piece of laboratory glass- or plastic-ware optionally comprising a cap on its upper end. The container comprising an opening for dispensing/ aspirating liquid into respectively out of the vessel. The opening may be closed by a cap, a breakable seal or like suitable means for closing the opening in a liquid-tight manner. Sample tubes, e.g. sample tubes used to collect blood, often comprise additional substances such as clot activators or anticoagulant substances which have an impact on the processing of the sample. As a consequence, different tube types typically are adapted for pre-analytical and analytical requirements of a particular analysis, e.g. a clinical chemistry analysis, a hematological analysis or a coagulation analysis. A mix up of sample tube types can make (blood) samples unusable for analysis. To prevent errors in the collection and handling of samples, the sample caps of many tube manufacturers are encoded according to a fixed and uniform color scheme. Some sample tubes types in addition or alternatively are characterized by particular tube dimensions, cap dimensions, and/or tube color. A dimension of a tube comprises e.g. its height, its size and/or further characteristic shape properties. Sample containers are identified using identification tag(s) attached thereto. The term 'identification tag' as used herein refers to an optical and/or radio frequency based identifier that allows the identifier tag to be uniquely identified by a corresponding identification tag reader.

The 'identification tag' shall comprise - but is not limited to - a barcode, a QR code or an RFID tag.

The expression 'tube type' refers to a category of sample tubes which can be characterized by at least one shared property, whereby said shared property can be automatically detected by a lab-device and can thus be used to discriminate a set of sample tubes of a first tube type from another. Some tube types are designed for carrying samples which can be used for a plurality of different analytical tests. An example for such a tube type is a serum tube. However, a tube type may also be particular for one single analytical test.

The term 'sample carrier' as used herein refers to any kind of holder configured to receive one or more sample tubes and configured to be used for transporting sample tube(s). Sample carriers may be of two major types, single holders and sample racks.

A 'single holder' is a type of sample carrier configured to receive and transport a single sample tube. Typically, a single holder is provided as a puck, i.e. a flat cylindrical object with an opening to receive and retain a single sample tube.

A 'sample rack' is a type of sample carrier, typically made of plastics and/or metal, adapted for receiving, holding and transporting sample tubes, e.g. 5 or more sample tubes e.g. disposed in one or more rows. Apertures, windows or slits may be present to enable visual or optical inspection or reading of the sample tubes or of the samples in the sample tubes or of a label, such as a barcode, present on the sample tubes held in the sample rack.

The term 'laboratory instrument' as used herein encompasses any apparatus or apparatus component operable to execute one or more processing steps / workflow steps on one or more biological samples and/or one or more reagents. The expression 'processing steps' thereby refers to physically executed processing steps such as centrifugation, aliquotation, sample analysis and the like. The term 'instrument' covers pre-analytical instruments, post-analytical instruments and also analytical instruments.

The term 'analyzer' / 'analytical instrument' as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'pre-analytical instrument' as used herein encompasses any apparatus or apparatus component that is configured to perform one or more pre-analytical processing steps / workflow steps comprising - but not limited to - centrifugation, resuspension (e.g. by mixing or vortexing), capping, decapping, recapping, sorting, tube type identification, sample quality determination and/or aliquotation steps. Said processing steps may also comprise adding chemicals or buffers to a sample, concentrating a sample, incubating a sample, and the like.

The term 'post-analytical instrument' as used herein encompasses any apparatus or apparatus component that is configured to perform one or more post-analytical processing steps / workflow steps comprising - but not limited to - sample unloading, transport, recapping, decapping, temporary storage/ buffering, archiving (refrigerated or not), retrieval and/ or disposal.

The term 'sample transportation system' as used herein encompasses any apparatus or apparatus component that is configured to transport sample carriers (each holding one or more sample containers) between laboratory instruments. In particular, the sample transportation system is a one dimensional conveyor-belt based system, a two-dimensional transportation system (such as a magnetic sample carrier transport system) or a combination thereof.

The term 'control unit' as used herein encompasses any physical or virtual processing device configurable to control a laboratory instrument / or system comprising one or more laboratory instruments in a way that workflow(s) and workflow step(s) are conducted by the laboratory instrument / system. The control unit may, for example, instruct the laboratory instrument / system to conduct pre-analytical, post analytical and analytical workflow(s)/ workflow step(s). The control unit may receive information from a data management unit regarding which steps need to be performed with a certain sample. In some embodiments, the control unit might be integral with a data management unit, may be comprised by a server computer and/or be part of one laboratory instrument or even distributed across multiple instruments of the analytical laboratory. The control unit may, for instance, be embodied as a programmable logic controller running a computer-readable program provided with instructions to perform operations.

A 'data management unit' or 'database' is a computing unit for storing and managing data. This may involve data relating to biological sample(s) to be processed by the automated system. The data management unit may be connected to an LIS (laboratory information system) and/or an HIS (hospital information system). The data management unit can be a unit within or co-located with a laboratory instrument. It may be part of the control unit. Alternatively, the database may be a unit remotely locater. For instance, it may be embodied in a computer connected via a communication network.

The term 'server' as used herein encompasses any physical machine or virtual machine having a physical or virtual processor, capable of accepting requests from and giving responses accordingly. It shall be clear to a person of ordinary skill in the art of computer programming that the term machine may refer to a physical hardware itself, or to a virtual machine such as a JAVA Virtual Machine JVM, or even to separate virtual machines running different Operating Systems on the same physical machine and sharing that machine's computing resources. Servers can run on any computer including dedicated computers, which individually are also often referred to as 'the server' or shared resources such as virtual servers. In many cases, a computer can provide several services and have several servers running. Therefore, the term server shall encompass any computerized device that shares a resource with one or more client processes.

The term 'communication network' as used herein encompasses any type of wireless network, such as a WiFi™, GSM™, UMTS or other wireless digital network or a cable based network, such as Ethernet™ or the like. In particular, the communication network can implement the Internet protocol (IP). For example, the communication network comprises a combination of cable-based and wireless networks.

The term 'user interface' as used herein encompasses any suitable piece of software and/or hardware for interactions between an operator and a machine, including but not limited to a graphical user interface for receiving as input a command from an operator and also to provide feedback and convey information thereto. Also, a system /device may expose several user interfaces to serve different kinds of users/ operators.

An 'analytical laboratory' as used herein comprises a control unit operatively coupled to one or more analytical; pre- and post-analytical work cells wherein the control unit is operable to control the instruments. In addition, the control unit may be operable to evaluate and/or process gathered analysis data, to control the loading, storing and/or unloading of samples to and/or from any one of the analyzers, to initialize an analysis or hardware or software operations of the analysis system used for preparing the samples, sample tubes or reagents for said analysis and the like. In particular, the instruments of an analytical laboratory and the control unit are interconnected by a communication network.

A 'test order' as used herein encompasses any data object, computer loadable data structure, modulated data representing such data being indicative of one or more processing steps to be executed on a particular biological sample. For example, a test order may be a file or an entry in a database. A test order can indicate an analytical test if, for example, the test order comprises or is stored in association with an identifier of an analytical test to be executed on a particular sample.

A 'STAT sample' is a sample which needs to be processed and analyzed very urgently as the analysis result may be of life-crucial importance for a patient.

The term 'workflow' as used herein refers to a collection of workflow steps /processing steps. According to particular embodiments, the workflow defines a sequence in which the processing steps are carried out.

The term 'workflow step' as used herein encompasses any activity belonging to a workflow. The activity can be of an elementary or complex nature and is typically performed at or by means of one or more work cell(s).

Embodiments of the disclosed method/ instrument shall now be described with reference to the figures.

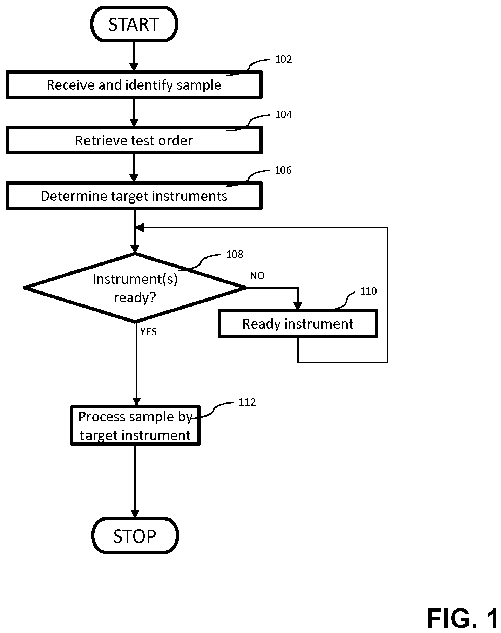

Figure 1 shows a flowchart illustrating a first embodiment of a herein disclosed method of operating an analytical laboratory. According to the method disclosed herein, in a first step 102, biological sample(s) are received and identified by a pre-analytical laboratory instrument of the analytical laboratory. The identification is performed in particular by an identifier tag reader reading an identifier tag attached to a sample container holding the biological sample.

In a subsequent step 104, an order list is retrieved from a database, the order list comprising one or more test order(s) defining at least one processing step to be carried out on the biological sample. Thereafter, in a step 106, a target laboratory instrument of the analytical laboratory is determined for each of the test order(s). In this determination, am analytical instrument is selected which is configured to carry out the at least one processing step according to the test order.

Once the target laboratory instrument has been determined, in a step 108, its availability is determined, in particular whether the target laboratory instrument(s) is ready/ ready to carry out the test order. Such may be based on a continuous inventory of laboratory resources and/or based on a query of the target laboratory instrument(s). The terms 'ready' or 'available' - in the context of the present application - is to be understood to comprises one or more of the following:
- The analytical instrument is switched on and not in a low power mode;
- All modules of the analytical instrument required to carry out the respective test order are operational;
- All consumables required to carry out the respective test order are available;
- All quality control and/or calibration steps required before carrying out the respective test order are available are up-to-date and valid.

If it has been determined (in step 108) that the target laboratory instrument is not ready to carry out the test order, in a subsequent step 110, a readiness command is transmitted to the target laboratory instrument(s). The readiness command comprises an instruction, which - when executed by the target laboratory instrument- causes the target laboratory instrument to become ready to carry out the test order. Upon receipt of the readiness command, still part of step 110, the target laboratory instrument executes the instruction(s) comprised therein in order to cause the target laboratory instrument to become ready to carry out the test order. Corresponding to the listing of the preceding paragraph, such includes:
- Waking up the analytical instrument from a low power mode;
- Ensuring all modules of the analytical instrument required to carry out the respective test order are operational;
- Making all consumables required to carry out the respective test order available;
- Performing all quality control and/or calibration steps required before carrying out the respective test order.

After the readiness of the target laboratory instrument to carry out the test order has been verified, in step 112, the target laboratory instrument is instructed to process the biological sample according to the test order. Still part of step 112, the target laboratory instrument(s) processes the biological sample according to the test order as instructed.

According to embodiments disclosed herein, the readiness command to the target laboratory instrument(s) is generated considering a readiness time of the target laboratory instrument to ensure it is ready to process the biological sample without delays. Corresponding to the broad interpretation of the term 'readiness', the term 'readiness time' shall be interpreted to include the amount of time required to carry out all activities covered by the process to make the instrument ready (such as wake-up from low power modes, ensuring all required modules are operational, performing quality control/ calibration steps if needed).

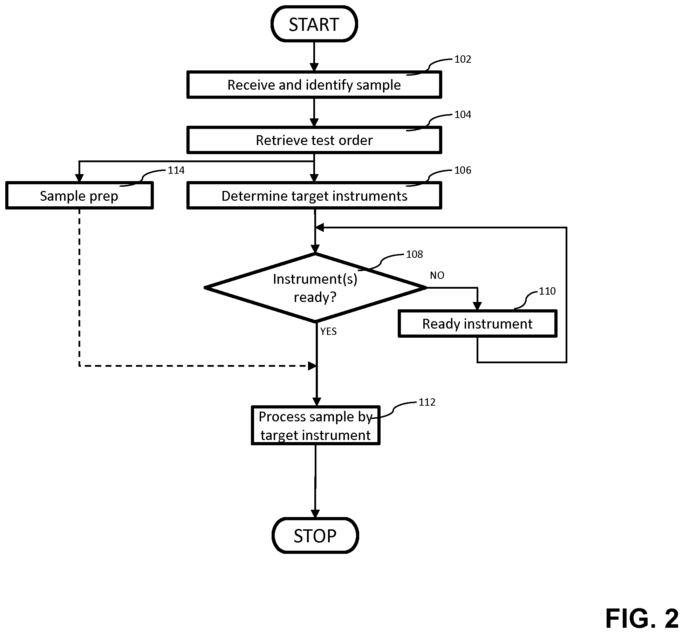

Turning now to figure 2, a further embodiment of a herein disclosed method of operating an analytical laboratory shall be described. As illustrated on the flowchart of this figure, in a step 114, the biological sample is processed by a pre-analytical laboratory instrument (also referred to as sample prep.). According to embodiments disclosed herein - such as illustrated on figure 2, the readiness command to the target laboratory instrument(s) is generated further considering a processing time of the biological sample by the pre-analytical laboratory instrument. The processing time relates to the time required to carry out the sample preparatory steps (such as centrifugation, resuspension - e.g. by mixing or vortexing), capping, decapping, recapping, sorting, tube type identification, sample quality determination and/or aliquotation) required to be performed before the biological sample can be processed by the target laboratory instrument(s).

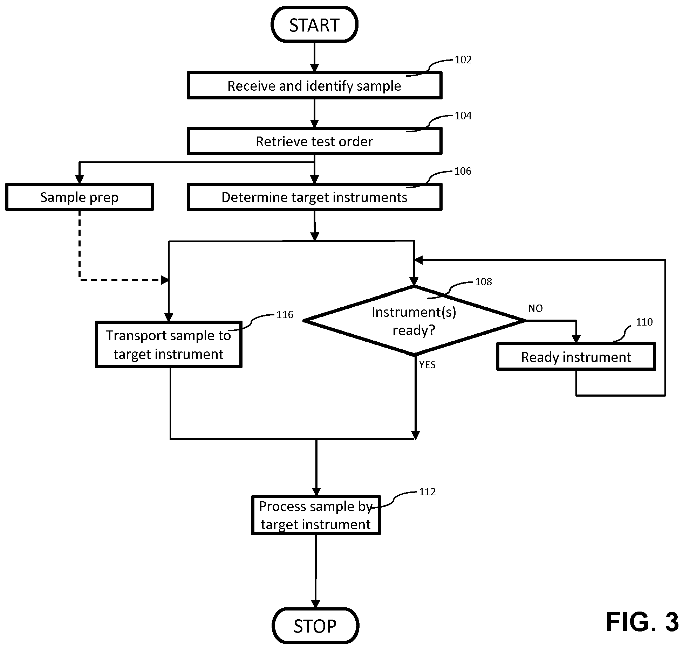

Figure 3 shows a flowchart of a further embodiment of a herein disclosed method of operating an analytical laboratory, which - in addition to a pre-analytical laboratory instrument for sample preparation - further comprises a sample transportation system at least for transporting the biological sample from the pre-analytical laboratory instrument to the analytical instrument. According to embodiments disclosed herein - such as illustrated on figure 3, the readiness command to the target laboratory instrument(s) is generated further considering a transportation time of the biological sample from the pre-analytical laboratory instrument to the target laboratory instrument by a sample transportation system of the analytical laboratory.

All-in-all the timing and generation of the readiness command - taking the above parameters in consideration - is aimed at ensuring that the target laboratory instrument becomes ready by the time the biological sample is processed by the pre-analytical laboratory instrument and/or has been transported to the target laboratory instrument by a sample transportation system. In this way a so-called just in time availability of the analytical instruments can be ensured to maximize the use of low-power modes thereof while avoiding delays in the processing of the biological samples and hence of the analytical results obtained.

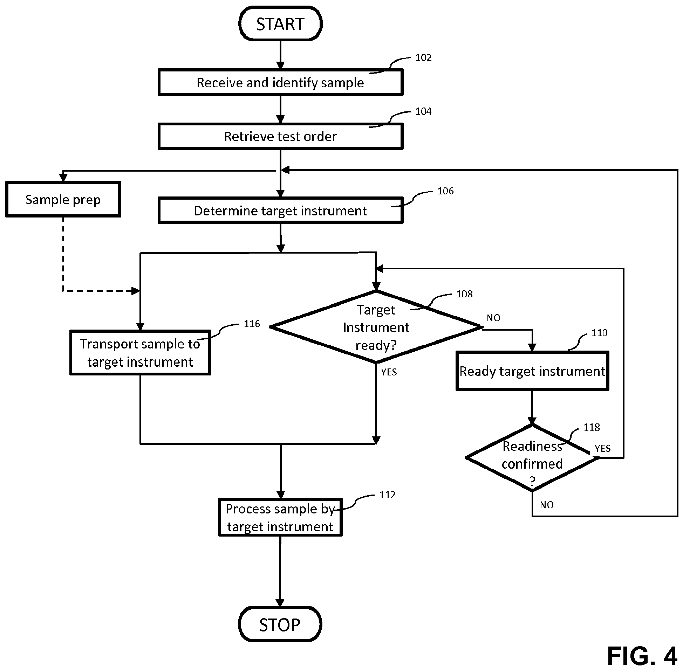

In order to provide an additional layer of certainty to the Turn-Around-Time and to potentially avoid unnecessary delays, embodiments disclosed herein - such as shown on figure 4 - comprise a check (using command-response) whether the target instrument is actually able become ready to perform the test order on the biological sample. The readiness confirmation, step 118, comprises two substeps. In a first substep, the target laboratory instrument transmits a readiness response, the readiness response being indicative whether the target laboratory instrument is able to execute the readiness command. According to further embodiments disclosed herein, the readiness response further comprises a readiness complete time indicative of the time forecast to be required for the analytical instrument to become ready to process the biological sample according to the respective test order. In a second substep, if the readiness response by the target laboratory instrument indicates that the target laboratory instrument is not able to execute the readiness command, an alternative target laboratory instrument is searched for. The search for an alternative target laboratory instrument is performed by repeating steps 106 through 110 until an alternative target laboratory instrument is determined which confirms by a readiness response that it is able to execute the readiness command.

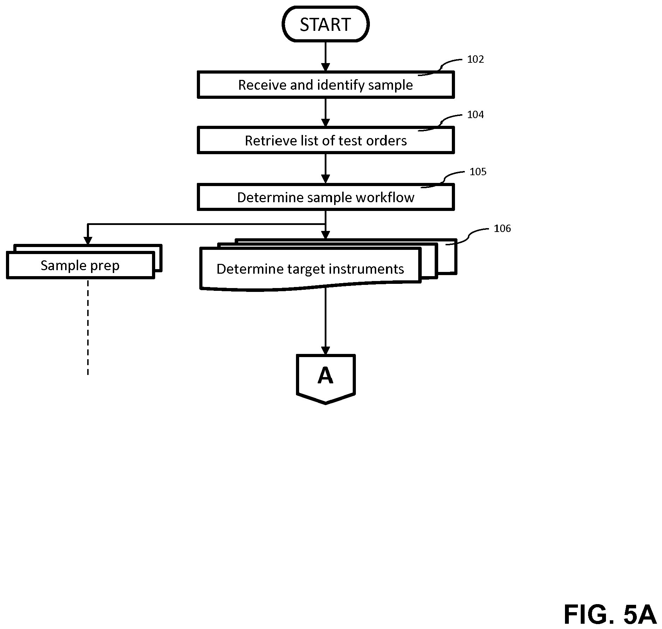

Embodiments of the disclosed method/ system shall be described with reference to figures 5A through 5D which comprise a plurality of laboratory instruments and the order list corresponding to a biological sample comprising a plurality of test orders.

Figure 5A shows the first page of a flowchart illustrating a first embodiment of a herein disclosed method of operating an analytical laboratory comprising a plurality of laboratory instruments. In addition to the steps already described with reference to the previous figures, figure 5A shows step 105 of determining a sample workflow for processing the biological sample comprising a sequence of workflow steps corresponding to each test order of the order list. According to embodiments of the disclosed method/system, the sample workflow defines one or more of:
- A number n of aliquots to be prepared from the biological sample.
   This number n defines into how many portions the biological sample is divided, such portions being referred to as aliquots of the biological sample. The biological sample as received is then referred to as primary sample. Depending on the test orders, the number of aliquots might be even 0 - in other words no aliquots are created and all processing steps of all targets are performed on the primary sample. Such workflow is also referred to as aliquotless workflow.
- Allocation of an aliquot or primary sample to each target.
   Once the required number n of aliquots has been determined (could be 0 as mentioned above), an aliquot or the primary sample is allocated to each target for performing the one or more processing steps at said target.
- Sequence in which the targets are to be processed.
   The target processing sequence defines the order in which the biological sample is processed by the targets. The sequence is of great importance especially when the same aliquot or primary sample is processed by more than one laboratory instruments of different sensitivity and/or contamination risk.
- Timing of processing of the targets
   According to embodiments of the disclosed method/ system, in addition to the processing sequence, the timing according to which the biological sample or aliquots thereof are processed is also defined by the sample workflow. For example, the timing is of great importance if the biological sample first needs to be prepared by a pre-analytical instrument and must be processed by an analytical instrument immediately thereafter. Another example is when the biological sample needs to spend a very specific amount of time in a pre-analytical instrument such as an incubator or centrifuge to ensure proper sample preparation for an analytical instrument. Furthermore, the timing of the processing of the targets is also relevant in view of sample degradation which is often correlated with its processing time, especially when the sample is outside of a temperature controlled area, in which case the sample should be transferred to a post-analytical instrument such as a temperature - controlled archiving unit after a certain amount of time. Another example is when certain processing steps, in particular certain rarely performed analytical tests are performed relatively rarely in an analytical laboratory. In such cases, embodiments of the disclosed method/ system align the timing of processing of the targets with a schedule of tests performed by the analytical laboratory in order to avoid that the respective target cannot be performed for an extended period of time.
   Timing of processing of the targets is also of great importance in view of the validity of quality control and/or calibration of certain laboratory instruments, in particular analytical instruments.

Once the sample workflow is determined, in a subsequent step 106, target instruments are determined for each of the test order(s) which is configured to carry out the at least one processing step according to the test order. Thereafter a plurality of readiness commands is generated for each target laboratory instrument, each readiness command for a particular target laboratory instrument.

In order to ensure just-in-time availability of the instruments, each readiness command for a particular target laboratory instrument is determined considering:
- a sum of transportation time of the biological sample from the pre-analytical laboratory instrument to each target laboratory instrument; and
- a sum of processing time of the biological sample by the pre-analytical laboratory instrument and each target laboratory instrument preceding the particular target laboratory instrument in the sample workflow.

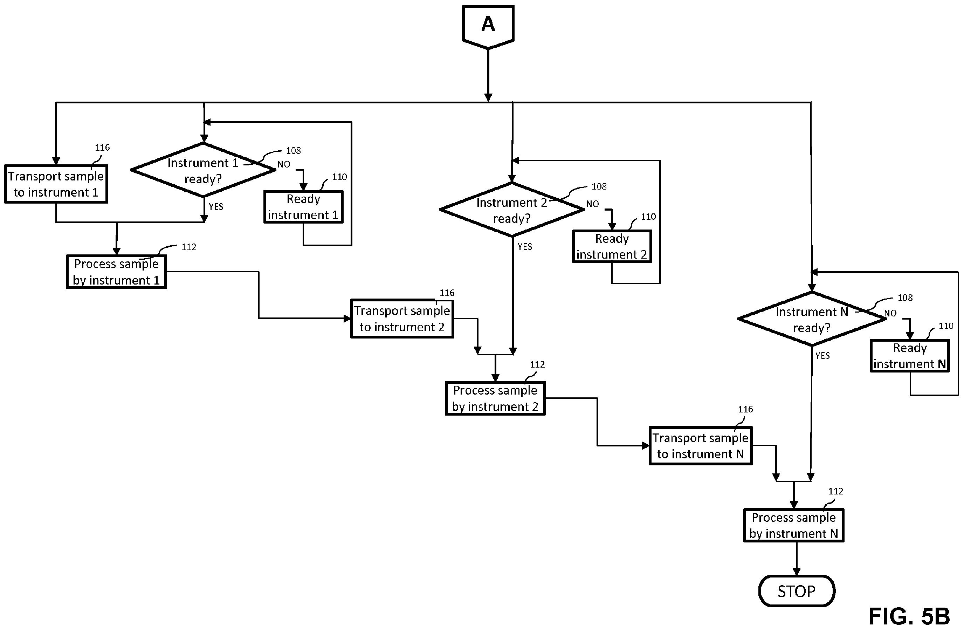

Figure 5B shows the second page of a flowchart illustrating the timing of the readiness command to the plurality of analytical laboratory instruments, such timing being in sync with the transport time and processing time of the biological sample by instruments respectively transportation system.

According to embodiments disclosed herein, the readiness response by the target laboratory instruments comprise an indication of an estimated time of availability of when the target laboratory instrument will become ready to carry out the respective test order.

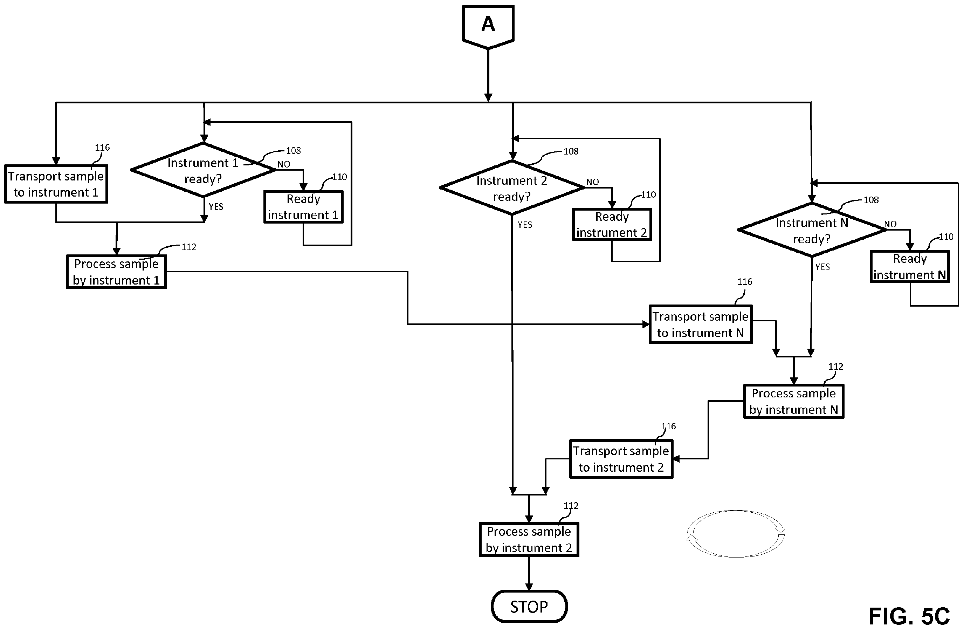

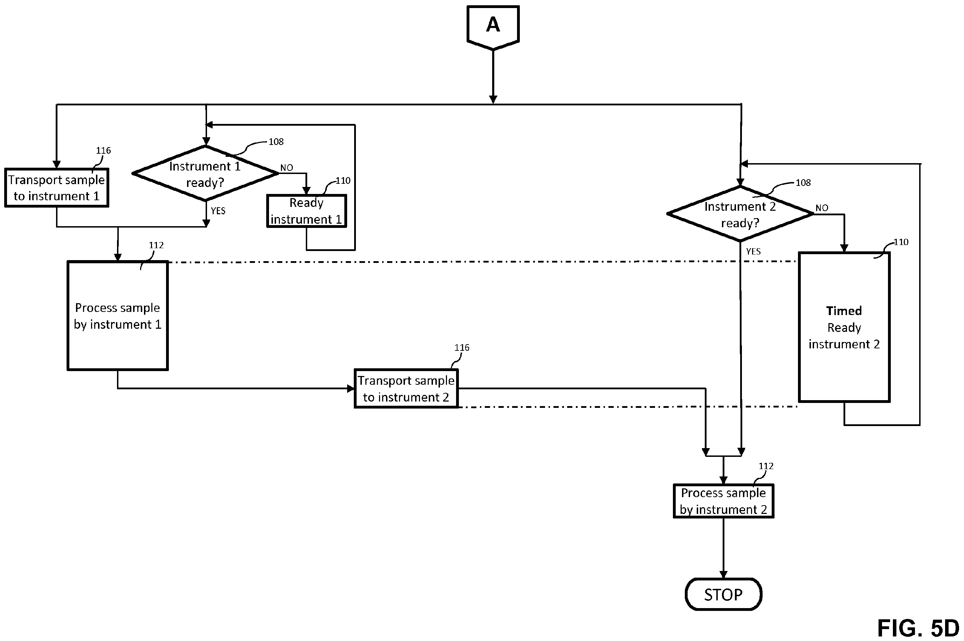

Figure 5C shows the second page of a flowchart illustrating embodiments of a herein disclosed method wherein the sample workflow is adapted as a reaction to the readiness respectively readiness time of an target laboratory instrument. In particular, figure 5 illustrates how the sample workflow for processing the biological sample is re-determined by postponing processing of the biological sample by a first target laboratory instrument if a second target laboratory instrument will become ready earlier to carry out its corresponding test order. It shall be noted that such re-determination of the sample workflow is possible if further constraints on the order of processing are not violated (e.g. cross-contamination risks from a less sensate instrument followed by a more sensitive analytical test).

Fig. 5D shows the second page of a flowchart illustrating embodiments of a herein disclosed method wherein the readying of a particular target laboratory instrument is delayed in view of processing time of the sample prior to processing by the particular analytical laboratory. Alternatively, or additionally the readiness command is transmitted to the analytical instrument as soon as generated and at the same time the readiness command comprises a delay time or readiness time.

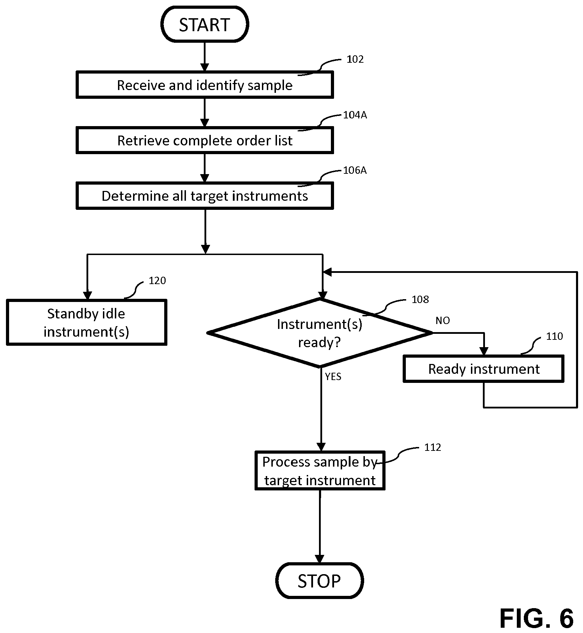

Turning now to figure 6, further embodiments of the disclosed method are described which - in addition to readying instruments on-demand - also comprise steps aimed at instructing laboratory instruments to switch into a low-power mode. In order to achieve this aim, in a step 104A, a complete order list is retrieved from a database, the complete order list comprising all test order(s) registered in the database that have not been completed. It must be emphasized that the complete order list comprises orders for all samples and not only the orders corresponding to the sample received and identified in step 102. In a subsequent step 106A, a target laboratory instrument of the analytical laboratory which is configured to carry out the at least one processing step according to the test order is identified for each of the test order(s) of the complete order list. Thereafter, in a step 110, a standby command is transmitted to any analytical instrument of the analytical laboratory not identified as target laboratory instrument for any of the test order(s) of the complete order list. In response to the standby command, analytical instrument(s) switch into a low power mode. Low power mode comprises any operational mode of an instrument which saves a resource, in particular electricity or consumable(s), including but not limited to stand-by or hibernation modes or even complete shutdown.

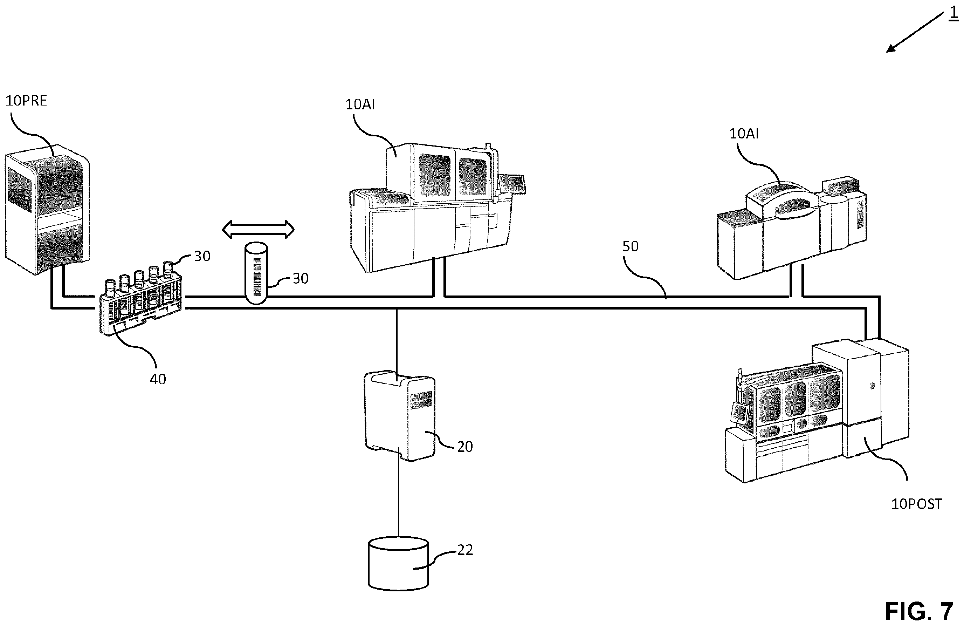

Fig. 7 shows a highly schematic block diagram of an embodiment of the disclosed analytical laboratory 1. As shown on the block diagram of figure 7, embodiments of the disclosed analytical laboratory 1 for processing biological sample(s) comprise a plurality of laboratory instruments 10, 10PRE, 10LD, 10AI, 10PA and a control unit 20 communicatively connected by a communication network. The plurality of laboratory instruments 10, 10PRE, 10LD, 10AI, 10PA are configured to execute processing steps on the biological samples according to instructions from the control unit 20.

The pre-analytical instruments 10PRE comprised by the analytical laboratory 1 may be one or more from the list comprising: an instrument for centrifugation of samples, a capping-, decapping- or recapping instrument, aliquoter, a buffer to temporarily store biological samples or aliquots thereof.

The post-analytical instruments 10POST comprised by the analytical laboratory 1 may be one or more from the list comprising: a recapper, an unloader for unloading a sample from an analytical system and/or transporting the sample to a storage unit or to a unit for collecting biological waste.

According to various embodiments of the disclosed analytical laboratory 1, the plurality of laboratory instruments 10, 10PRE, 10LD, 10AI, 10PA may be identical or different instruments such as clinical- & immunochemistry analyzers, coagulation chemistry analyzers, immunochemistry analyzers, urine analyzers, nucleic acid analyzers, hematology instruments etc.

The control unit 20 is configured to control the laboratory system 1 to carry out the steps of one or more of the methods herein disclosed.

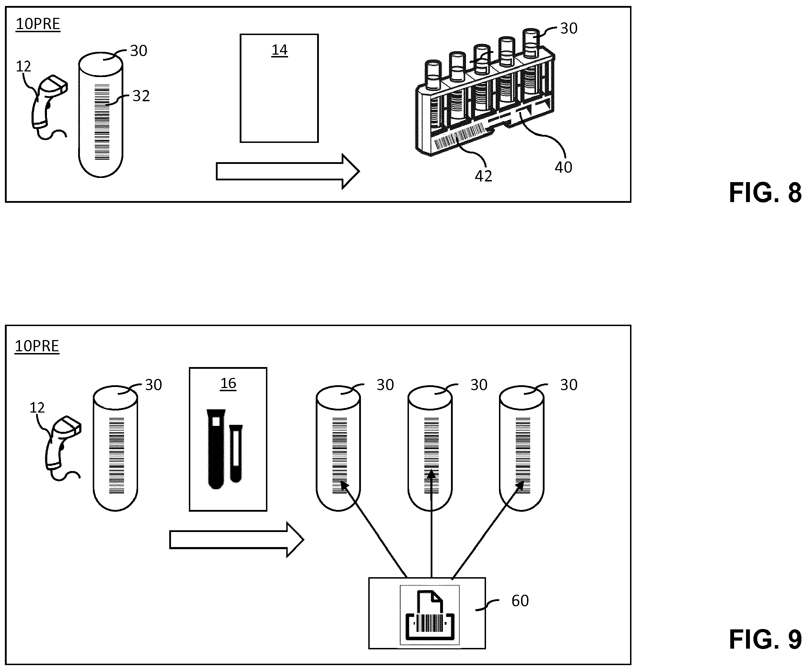

Turning now to figures 8 through 11, particular embodiments of the laboratory instruments 10PRE, 10POST, 10AI are described.

Figure 8 shows a pre-analytical laboratory instrument 10PRE comprising a sample container sorting unit 14 configured to sort sample containers 30 holding biological samples into sample carriers 40, each sample carrier 40 being identified by means of a carrier identifier (Carrier-ID) of a carrier tag 42 attached to the sample carrier 40, the pre-analytical laboratory instruments 10PRE being further configured to transmit signals to the laboratory control unit associating the sample identifier(s) ID of sorted sample containers 30 with the sample carrier identifier(s) Carrier-ID of the corresponding sample carrier(s) 40. For embodiments where a pre-analytical laboratory instrument 10PRE sorts sample containers 30 into sample carriers 40, one or more analytical laboratory instruments are further configured to read the carrier identifier Carrier-ID from the carrier tag 42 and transmit said carrier identifier Carrier-ID to the laboratory control unit with the test query.

Figure 9 shows a further embodiment of a pre-analytical laboratory instrument 10PRE, comprising an aliquoting unit 16 configured to prepare aliquots of biological sample(s) from the sample container(s) 30 and provide each of said aliquots with a sample identifier ID on an identifier tag 32 by means of an identifier tag writer 60.

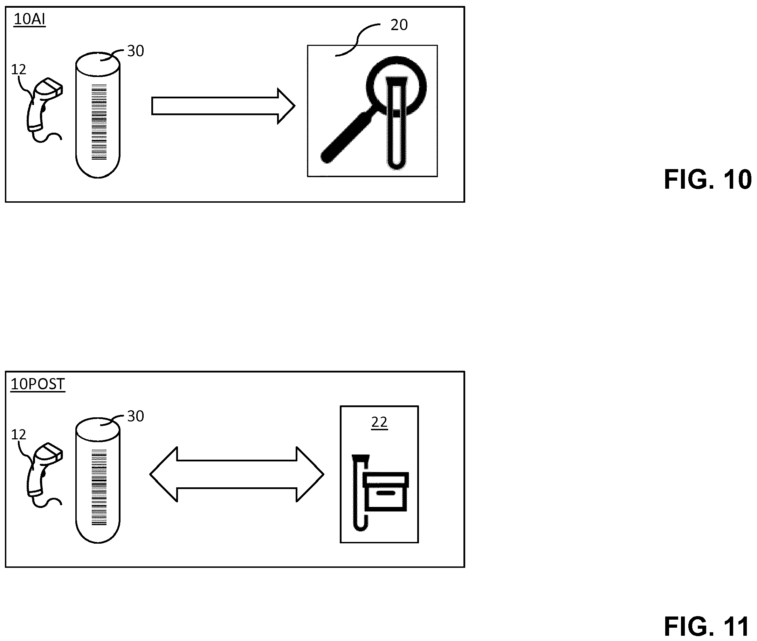

Figure 10 shows an embodiment of an analytical laboratory instrument 10AI, comprising an analytical unit 18 configured to carry out an analytical test to measure the presence and/or concentration of at least one analyte in the biological sample. The analytical laboratory instrument 10AI performs analytical test(s) of the biological sample in response to the test order(s).

Figure 11 shows an embodiment of a post-analytical laboratory instrument 10POST comprising a storage unit 19. The post-analytical laboratory instrument 10AI is configured to store respectively retrieve sample containers 30 into respectively from the storage unit 19. The query by post-analytical laboratory instrument(s) 10POST to the laboratory control unit for a processing order comprises a container to store respectively retrieve into respectively from the storage unit 19. Correspondingly, when queried by a post-analytical laboratory instrument 10POST, the control unit transmits data indicative of a sample container 30 to be retrieved from the storage unit 19. In response to the data indicative of a sample container 30 to be stored respectively retrieved, the post-analytical laboratory instrument 10POST stores respectively retrieves the sample container 30 from the storage unit 19.

Further disclosed and proposed is a computer program product including computer-executable instructions for performing the disclosed method in one or more of the embodiments enclosed herein when the program is executed on a computer or computer network. Specifically, the computer program may be stored on a computer-readable data carrier or a server computer. Thus, specifically, one, more than one or even all of method steps as indicated above may be performed by using a computer or a computer network, preferably by using a computer program.

As used herein, a computer program product refers to the program as a tradable product. The product may generally exist in any format, such as in a downloadable file, on a computer-readable data carrier on premise or located at a remote location (cloud). Specifically, the computer program product may be distributed over a data network (such as a cloud environment). Furthermore, not only the computer program product, but also the execution hardware may be located on-premise or in a cloud environment.

Further disclosed and proposed is a computer-readable medium comprising instructions which, when executed by a computer system, cause an analytical laboratory to perform the method according to one or more of the embodiments disclosed herein.

Further disclosed and proposed is a modulated data signal comprising instructions which, when executed by a computer system, cause an analytical laboratory to perform the method according to one or more of the embodiments disclosed herein.

**REFERENCE LIST:**

| | |
|---|---|
| analytical laboratory | 1 |
| laboratory instrument | 10 10PRE, 10POST, 10AI |
| pre-analytical laboratory instrument | 10PRE |
| analytical laboratory instrument | 10AI |
| post-analytical laboratory instrument | 10POST |
| identifier tag reader | 12 |
| sample container sorting unit | 14 |
| aliquoting unit | 16 |
| analytical unit | 20 |
| storage unit | 22 |
| sample container | 30 |
| identifier tag | 32 |
| sample carrier | 40 |
| carrier tag | 42 |
| sample transportation system | 50 |
| identifier tag writer | 60 |
| receive and identify sample | step 102 |
| retrieve test order(s) | step 104 |
| retrieve complete order list | step 104A |
| determine sample workflow | step 105 |
| determine target laboratory instrument | step 106 |
| determine all target instruments | step 106A |
| determine target laboratory instrument readiness | step 108 |
| ready analytical instrument | step 110 |
| process sample by target instrument | step 112 |
| sample prep by pre-analytical laboratory instrument | step 114 |
| transport sample to target instrument | step 116 |
| readiness confirmation | step 118 |
| standby idle instrument(s) | step 120 |

## Claims

1. A method of operating an analytical laboratory (1), comprising the steps of:
a) receiving and identifying a biological sample by a pre-analytical laboratory instrument (10PRE) of the analytical laboratory (1);
b) retrieving an order list from a database (22), the order list comprising one or more test order(s) defining at least one processing step to be carried out on the biological sample;
c) determining a target laboratory instrument of the analytical laboratory (1) for each of the test order(s) which is configured to carry out the at least one processing step according to the test order;
d) determining whether the target laboratory instrument(s) is ready to carry out the test order;
e) transmitting a **readiness command** to the target laboratory instrument(s) if the target laboratory instrument(s) is not ready to carry out the test order, the readiness command comprising an instruction, which - when executed by the target laboratory instrument - causes the target laboratory instrument to become ready to carry out the test order;
f) executing instructions of the readiness command by the target laboratory instrument in order to cause the target laboratory instrument to become ready to carry out the test order;
g) instructing the target laboratory instrument(s) to process the biological sample according to the test order if the target laboratory instrument is ready to carry out the test order
h) the target laboratory instrument(s) processing the biological sample according to the test order as instructed.

2. A method of operating an analytical laboratory (1) according to claim 1, wherein the readiness command is generated considering:
- a processing time of the biological sample by the pre-analytical laboratory instrument (10PRE) and/or
- a transportation time of the biological sample from the pre-analytical laboratory instrument (10PRE) to the target laboratory instrument by a sample transportation system (50) of the analytical laboratory (1); and/or
- a readiness time of the target laboratory instrument,
such that the target laboratory instrument becomes ready by the time the biological sample is processed by the pre-analytical laboratory instrument (10PRE) and/or has been transported to the target laboratory instrument by a sample transportation system (50).

3. A method of operating an analytical laboratory (1) according to claim 1 or 2, further comprising the steps of:
e') transmitting a readiness response by the target laboratory instrument, the readiness response being indicative whether the target laboratory instrument is able to execute the readiness command;
e") if the readiness response by the target laboratory instrument indicates that the target laboratory instrument is not able to execute the readiness command, repeating steps c) through e) until an alternative target laboratory instrument is determined which confirms by a readiness response that the target laboratory instrument is able to execute the readiness command.

4. A method of operating an analytical laboratory (1) according to one of the claims 1 to 3, wherein the order list comprises a plurality of test orders, each test order defining at least one processing step to be carried out on the biological sample, the method further comprising the steps:
- determining a sample workflow for processing the biological sample comprising a sequence of workflow steps corresponding to each test order of the order list;
- generating a plurality of readiness commands for each target laboratory instrument, each readiness command for a particular target laboratory instrument considering:
∘ a sum of transportation time of the biological sample from the pre-analytical laboratory instrument (10PRE) to each target laboratory instrument; and
∘ a sum of processing time of the biological sample by the pre-analytical laboratory instrument (10PRE) and each target laboratory instrument preceding the particular target laboratory instrument in the sample workflow.

5. A method of operating an analytical laboratory (1) according to claim 4, further comprising the steps:
- transmitting a readiness response by each target laboratory instrument, the readiness response being indicative of an estimated time of availability of when the target laboratory instrument will become ready to carry out the respective test order;
- re-determining the sample workflow for processing the biological sample by postponing processing of the biological sample by a first target analytical laboratory instrument (10PRE) if a second target analytical laboratory instrument (10PRE) will become ready earlier to carry out its corresponding test order.

6. A method of operating an analytical laboratory (1) according to one of the claims 1 to 5, wherein the step of determining whether the target laboratory instrument(s) is ready to carry out the test order comprises one or more of the following:
- determining whether the target laboratory instrument is powered on and not in a low-power mode;
- determining whether all modules of the target laboratory instrument required to carry out the respective test order are operational;
- determining whether all consumables required to carry out the respective test order are available;
- determining whether all quality control and/or calibration values of the target laboratory instrument are up-to-date and valid;
and wherein the readiness command comprises instructions, which - when executed by the target laboratory instrument - causes one or more of:
- powering on respectively waking up the target laboratory instrument from a low-power mode;
- all modules of the target laboratory instrument required to carry out the respective test order to be brought into operational state;
- all consumables required to carry out the respective test order to be made available to the target laboratory instrument;
- carrying out of quality control and/or calibration steps to ensure all quality control and/or calibration values of the target laboratory instrument are up-to-date and valid.

7. A method of operating an analytical laboratory (1) according to one of the claims 1 to 6, further comprising:
- retrieving a complete order list from a database (22), the complete order list comprising all test order(s) registered in the database (22) that have not been completed;
- identify a target laboratory instrument of the analytical laboratory (1) for each of the test order(s) of the complete order list which is configured to carry out the at least one processing step according to the test order;
- transmitting a standby command to any analytical instrument (10AI) of the analytical laboratory (1) not identified as target laboratory instrument for any of the test order(s) of the complete order list;
- target laboratory instrument switching into a low power mode in response to the standby command.

8. A method of operating an analytical laboratory (1) according to one of the claims 1 to 7, wherein one or more of the target laboratory instrument(s) is an analytical laboratory instrument (10AI) configured to carry out one or more analytical processing steps on the biological sample to determine presence and/or concentration of one or more analyte(s) in the biological sample.

9. An analytical laboratory (1) comprising:
- one or more pre-analytical laboratory instrument(s) (10PRE) configured to receive and identify biological sample(s);
- one or more laboratory instrument(s) configured to perform one or more processing step(s) on the biological sample;
- a control unit (20) communicatively connected to the pre-analytical laboratory instrument(s) (10PRE) and analytical instrument(s) (10AI), the control unit (20) being configured to control the analytical laboratory (1) to perform any of the methods of the preceding claims.

10. An analytical laboratory (1) according to claim 9, wherein the one or more laboratory instrument(s) comprise one or more analytical laboratory instrument (10AI) configured to carry out one or more analytical processing steps on the biological sample to determine presence and/or concentration of one or more analyte(s) in the biological sample.

11. An analytical laboratory (1) according to claim 9 or 10, wherein the one or more laboratory instrument(s) comprise one or more post-analytical laboratory instrument(s)(10POST) configured to perform one or more from the list comprising recapping, unloading, disposing and archiving of biological sample(s).

12. An analytical laboratory (1) according to one of the claims 9 to 11, wherein the one or more laboratory instrument(s) comprise a sample transportation system (50) configured to transport biological sample(s) from a first laboratory instrument (10PRE, 10AI, 10PA) to a second laboratory instrument (10PRE, 10AI, 10PA).

13. A computer program product comprising instructions which, when executed by a control unit (20) of an analytical laboratory (1), cause the analytical laboratory (1) to perform the steps of any one of the methods according to claims 1 through 8.
